# EUROPÄISCHE PATENTSCHRIFT

(11) **EP 1 633 705 B1**
(45) Veröffentlichungstag und Bekanntmachung des Hinweises auf die Patenterteilung: **19.06.2013**
(21) Anmeldenummer: 04724562.6
(22) Anmeldetag: 31.03.2004
(51) Int. Cl.: C07C 319/24

(54) **OXIDATION VON MERCAPTOETHANOL**
OXIDATION OF MERCAPTOETHANOL
OXYDATION DE MERCAPTOÉTHANOL

(30) Priorität: 23.05.2003 DE 10323839
(43) Veröffentlichungstag der Anmeldung: 15.03.2006
(73) Patentinhaber: Akzo Nobel N.V., 6824 BM Arnhem (NL)
(72) Erfinder: ZEITLER, Michael, 53347 Alfter (DE); KOTTNER, Nils, 78628 Rottweil (DE); BERGFELD, Manfred, 63906 Erlenbach-Mechenhard (DE)
(74) Vertreter: Akzo Nobel IP Department
(86) Internationale Anmeldenummer: PCT/EP2004/003383
(87) Internationale Veröffentlichungsnummer: WO 2004/103956

(56) Entgegenhaltungen:
- EP-A- 0 288 104
- US-A- 2 792 334
- US-A- 4 721 813
- US-A- 6 051 740
- CHOI, J. AND YOON, N.: "Synthesis of disulfides by copper-catalyzed disproportionation of thiols" J.ORG.CHEM., Bd. 60, 1995, Seiten 3266-3267, XP002289094

## Beschreibung

Die Erfindung betrifft ein Verfahren zur Herstellung von ' Dihydroxiethylendisulfid durch Umsetzung von Mercaptoethanol mit Sauerstoff oder sauerstoffhaltigen Gasen.

Diese Herstellung des Dihydroxiethylendisulfids kann durch folgende Reaktionsgleichung dargestellt werden.
2 HO - CH₂ CH₂ - SH + ½O₂→HO-CH₂CH₂-S-S-CH₂CH₂-OH + H₂O
Oxidationsreaktionen dieser Art sind bereits seit längerem bekannt. So wird in der US PS 3 978 137 ein Verfahren zur Oxidation von einer Schwefel enthaltenden Verbindung beschrieben, bei dem diese Verbindung mit einem Sauerstoff enthaltenden Gas in einem Medium umsetzt wird, welches einen pH-Wert im Bereich von 8 bis 14 aufweiset. Dabei wird ein Katalysatorsystem verwendet, das VII B Metallphthalocyanine und VIII Metallphthalocyanine enthält.

Den Beispiele ist zu entnehmen, dass hier Sulfide wie Natriumsulfid, Ammoniumsulfid, aber auch Thiophenyl und Methylmercaptan umgesetzt werden.

Auch werden in der Beschreibung innerhalb einer langen Auszählung allgemein Mercaptane mit zusätzlichen funktionellen Gruppen erwähnt wie z. B. Mercaptoethanol und Cystein. Konkrete Anweisungen zur Herstellung von Dihydroxiethylendisulfid aus Mercaptoethanol finden sich in dieser Patentschrift jedoch nicht.

Führt man Versuche zur Herstellung von Dihydröxiethylendisulfid durch Oxidation von Mercaptoethanol gemäß dem in dieser US-Patentschrift beschriebenen Verfahren durch, so sind zum einen sehr lange Reaktionszeiten notwendig, zum anderen sind die Ausbeuten an Dihydroxiethylendisulfid sehr gering.

Darüber hinaus entsteht aufgrund von Nebenreaktionen (Überoxidation) ein uneinheitliches Produkt, das aufwendig gereinigt werden muss. Auch ist das verwendete Katalysatorsystem sehr kompliziert, kostspielig, schwierig herzustellen und abzutrennen.

Auch das in der US-PS 4 090 954 beschriebene Verfahren zur Oxidation von Mercaptanen arbeitet mit speziellen, sehr komplizierten Katalysatoren nämlich Metallkomplexen auf Porphyrinbasis und ist für die Herstellung von Dihydroxiethylendisulfid wenig geeignet. Als komplexiertes Metall können die verschiedensten Metallen fungieren; einfache Metallsalze als Katalysator werden jedoch nicht erwähnt. Außerdem ist in dieser Patentschrift die Oxidation von Mercaptoethanol zu Dihydroxiethylendisulfid explizit nicht erwähnt.

In der US-PS 4 258 212 ist ein Verfahren zum Oxidieren von 2-Mercaptoethanol beschrieben, bei dem Wasserstoffperoxid als Oxidationsmittel verwendet wird, dabei entsteht jedoch ein Gemisch aus Di- und Trisulfiden. Außerdem wird Wasserstoffperoxid in überstöchiometrischen Mengen benötigt, was das Verfahren erheblich verteuert.

In der US-PS 4 288 627 wird ein Verfahren zur Herstellung von Disulfiden mittels Oxidation von Thiolen beschrieben, bei dem ein Katalysatorgemisch von Kobaltmolybdat und flüssigen tertiären Aminen verwendet wird. Die Herstellung von Dihydroxiethylendisulfid wird in dieser Patentschrift nicht beschrieben. Das Verfahren, übertragen auf die Herstellung von Dihydroxiethylendisulfid, arbeitet wenig selektiv und mit geringer Ausbeute, da es ebenfalls zu Nebenprodukten und Überoxidation führt

In der US PS 4 078 992 wird ebenfalls ein Verfahren beschrieben, bei dem mercaptanhaltige Kohlenwasserstoffdestillate mit Sauerstoff oder einem Sauerstoff enthaltenden Gas oxidiert werden, dabei wird als Katalysator ein Metallphthalocyanin eingesetzt.

Zusätzlich zu den dort beschriebenen Katalysatoren kann bei der Reaktion auch ein Promotor mitverwendet werden, z. B. Borsäure, Ammoniumchromat, Ammoniumchlorid und auch ein Metallsalz nämlich Eisen-III-clorid. Weitere Metallsalze als Promotor werden dort nicht explizit erwähnt.

Schließlich sei auf die US PS 4 721 813 verwiesen, welche die Herstellung von Disulfiden aus den entsprechenden Mercaptoalkanolen durch Oxidation mit Sauerstoff beschreibt. In dieser Patentschrift wird die Verwendung von wasserfreiem gasförmigem Ammoniak in geringen Mengen vorgeschrieben, nämlich in einem Mol-Verhältnis von Ammoniak zu Mercaptoalkanol unterhalb von 0,01 : 1. Außerdem ist zwingend vorgeschrieben, in Abwesenheit von jeglichen Metall enthaltenden Katalysatoren zu arbeiten. Zwar wird von einer hundertprozentigen Umsetzung geschrieben. Dazu ist allerdings ein verhältnismäßig hoher Druck erforderlich. Das erhaltene Produkt ist aber nicht geruchsfrei, außerdem besteht sehr leicht die Gefahr der Überoxidation.

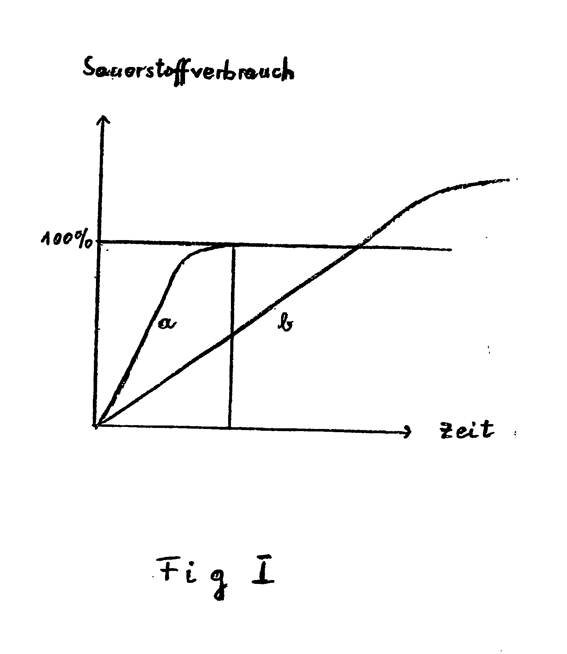

In Figur 1 wird der Verlauf einer Oxidation von Mercaptoethanol gemäß dem Verfahren nach der US PS 4 721 813 (Kurve b) schematisch mit dem Verlauf der Oxidation von Mercaptoethanol gemäß der Erfindung (Kurve a) gegenübergestellt. Die Kurve a zeigt, dass bei 100 % entsprechend dem theoretischen Verbrauch gemäß der eingangs angegebenen Reaktionsgleichung kein weiterer Sauerstoff mehr aufgenommen wird, die Reaktion also beendet ist und weitere Oxidationsreaktionen wie Überoxidation nicht stattfinden.

Die Kurve b zeigt, dass auch nach Verbrauch von 100 % der theoretischen Menge Sauerstoff weiterer Sauerstoff umgesetzt wird, was bedeutet, dass zusätzliche Reaktionen wie Überoxidation stattfinden. Es hat sich ferner herausgestellt, dass auch bereits, bevor die theoretische Menge von 100 % Sauerstoff verbraucht worden ist, Nebenrealdionen ablaufen, d. h. ein Teil des Sauerstoffs nicht entsprechend der eingangs angegebenen Reaktionsgleichung reagiert. Die Überoxidation macht sich durch Bildung von Verbindungen mit einer höheren Wertigkeit des Schwefels bzw. S-O Bindungen bemerkbar.

In der EP 0 288 104 wird ein Verfahren zur Herstellung von Disulfiden beschrieben, bei dem Thiole in einer alkalischen wässrigen Lösung in Gegenwart von organischen Lösungsmitteln oxidiert werden, die im Wesentlichen mit Wasser nicht mischbar sind. Bei den für die Oxidation vorgesehenen Thiole handelt es sich um übliche aliphatische zykloaliphatische oder aromatische Thiole, die auch Substituenten tragen wie Halogen andere aromatische oder aliphatische Kohlenwasserstoffegruppen, Alkoxygruppen oder Aryloxygruppen, Thiole, welche Hydroxigruppen tragen insbesondere Mercaptoethanol werden in dieser Schrift nicht genannt.

In J.ORG.CHEM., Bd.60, 1995, Seiten 3266 - 3267, wird die Disproportionierung von Thiolen in Gegenwart von Kupferkatalysatoren beschrieben. Die Disproportionierung findet unter Stickstoff statt. Eine Oxidation unter Verwendung von Oxidationsmitteln wird bei dem dort beschriebenen Verfahren nicht durchgeführt.

Wenn auch bereits eine Reihe von Verfahren bekannt sind, nach welchen Dihydroxiethylendisulfid durch Oxidation von Mercaptoethanol erhältlich ist, besteht immer noch ein starkes Bedürfnis nach verbesserten, technisch leicht realisierbaren, umweltfreundlichen und preiswerten Verfahren zur Herstellung von Dihydroxiethylendisulfid durch Umsetzung von Mercaptoethanol.

Aufgabe der Erfindung ist es deshalb ein Verfahren zur Verfügung zu stellen, das einfach durchzuführen ist, das zu einem einheitlichen Produkt mit definierter Zusammensetzung führt, das mit hoher Ausbeute und hoher Selektivität arbeitet und das innerhalb von kurzen Reaktionszeiten durchzuführen ist.

Aufgabe der Erfindung ist es ferner ein Verfahren zur Verfügung zu stellen, das wirtschaftlich durchzuführen ist und insbesondere keine Probleme für Abluft und Abwasser mit sich bringt und bei dem keine Gefahr der Überoxidation besteht, auch wenn ein Überangebot von Sauerstoff respektive sauerstoffhaltigem Gas vorhanden ist.

Diese Aufgabe wird gelöst durch ein Verfahren zur Herstellung von Dihydroxiethylendisulfid durch Umsetzung von Mercaptoethanol mit Sauerstoff oder sauerstoffhaltigen Gasen, bei dem Mercaptoethanol in Gegenwart von Ammoniak und/oder Aminen unter Verwendung von Kupfer- oder Mangansalzen mit Sauerstoff oder sauerstoffhaltigen Gasen umgesetzt wird.

Vorzugsweise werden als Katalysator Kupfer-(II) oder Mangan-(II-Salze) verwendet. Als Kupfer-(II)-salz ist Kupferacetat und als Mangan-(II)-salz Manganacetat besonders geeignet.

Es ist vorteilhaft, Ammoniak als wässrigen Ammoniak einzusetzen. Amine werden bevorzugt wasserfrei eingesetzt.

Vorzugsweise wird die Umsetzung bei 20 - 70° C insbesondere bei 35 - 45° C durchgeführt.

Es ist vorteilhaft, bei der Umsetzung Mercaptoethanol vorzulegen und Sauerstoff in die Vorlage zu dosieren.

Es ist ferner vorteilhaft, die Umsetzung unter einem Sauerstoffpartialdruck von 0,5 - 5 bar durchzuführen.

Bevorzugt wird die Reaktion unter einem Sauerstoffüberdruck, insbesondere bei einem Überdruck von 1 bis 5 insbesondere 1 - 2,5 bar durchgeführt.

Es ist vorteilhaft, die Reaktion unter einem konstantem Sauerstoffüberdruck durchzuführen.

Es ist vorteilhaft, das Verfahren ohne zusätzliches organisches Lösungsmittel durchzuführen.

In einer vorteilhaften Ausführungsform des erfindungsgemäßen Verfahrens wird die Umsetzung in wässrigem Milieu durchgeführt. Wässriges Milieu im Rahmen der Erfindung bedeutet, dass Wasser in Mengen vorhanden ist, die von wenigen Prozenten bis z. B. 70 % oder mehr, bezogen auf die Gesamtmenge der eingesetzten Stoffe, reichen.

Es ist vorteilhaft, wenn das Wasser in Mengen von 10 bis 70 Gew.-% vorhanden ist.

Es ist möglich beispielsweise das Wasser vorzulegen, sodann Mercaptoethanol zuzugeben, mit dem Katalysator sowie Ammoniak bzw. Aminen versetzen und sodann den Sauerstoff oder ein sauerstoffhaltiges Gas in den Reaktor einzuleiten.

Es ist auch möglich, Mercaptoethanol zunächst direkt in Wasser zu lösen und diese Lösung dem Reaktionsgefäß zuzuführen.

Durch die Mitverwendung von Wasser ist es möglich, die Geschwindigkeit der Umsetzung zu steuern, ohne die Ausbeute und Selektivität negativ zu beeinflussen. Dadurch wird auch die in der Zeiteinheit frei werdende Wärmemenge gesteuert und es kann ein zu schnelles Ansteigen der Temperatur oder ein zu Heftigwerden der Reaktion vermieden werden.

Die Arbeitsweisen mit Sauerstoff zusammen mit Inertgasen und/oder in wässrigem Milieu haben zudem sicherheitstechnische Vorteile. Dadurch werden nämlich mögliche Explosionsgefahren, die z. b. bei plötzlichem Auftreten einer Zündquelle entstehen können, vermieden.

Das erfindungsgemäße Verfahren kann beispielsweise wie folgt durchgeführt werden.

In einem Druckreaktor wird Mercaptoethanol vorgelegt und mit katalytischen Mengen wässrigem konzentriertem Ammoniak sowie katalytischen Mengen an Mangansalz, vorzugsweise Manganacetat oder entsprechenden katalytischen Mengen eines Kupfersalzes insbesondere Kupferacetat versetzt.

Anschließend wird Sauerstoff unter Druck beispielsweise mit 2,5 bar unter starkem Rühren eingeleitet. Dabei wird der Reaktor gekühlt. Anstelle von reinem Sauerstoff kann auch ein sauerstoffhaltiges Gasgemisch, wie z. B. Luft oder Sauerstoff und ein oder mehrere Inertgase wie Stickstoff oder Edelgase verwendet werden.

Um einen entsprechenden Sauerstoffdruck zu erhalten, muss selbstverständlich der Gesamtdruck so eingestellt werden, dass der gewünschte Sauerstoffpartialdruck erreicht wird.

Bevorzugt wird das Metallsalz in Ammoniak, bzw. in dem Anim gelöst zugesetzt.

Um eine vergleichbare Reaktionsgeschwindigkeit wie beim Arbeiten mit reinem Sauerstoff zu erreichen, muss bei Verwendung von sauerstoffhaltigem Gasgemisch natürlich der Gesamtdruck des Gasgemisches entsprechend erhöht werden, damit der Sauerstoffpartialdruck dem Druck des Sauerstoffs bei der Arbeitsweise mit reinem Sauerstoff entspricht.

Die Reaktion springt sofort an. Nach Möglichkeit sollte die Temperatur nicht über 50° C steigen. Die Reaktion ist stark exotherm, deshalb ist es zweckmäßig für eine entsprechende effiziente Kühlung zu sorgen. Sollte die Temperatur dennoch über 50° C steigen, kann durch Erniedrigung des Sauerstoffpartialdrucks die Reaktion abgebremst werden.

Der Sauerstoff kann direkt in das Mercaptoethanol eingeleitet werden, der sich sofort in der Flüssigkeit verteilt und reagiert. Es ist aber auch möglich, den Sauerstoff oberhalb der Flüssigkeitsoberfläche einzuleiten, dass sozusagen über dem Mercaptoethanol eine Sauerstoffatmosphäre entsteht. Während des Rührens (bevorzugt mit einem Begasungsrührer) nimmt das Mercaptoethanol aus der Atmosphäre schnell den benötigten Sauerstoff auf.

Die Reaktion ist beendet, wenn die exakt stöchiometrische Menge Sauerstoff verbraucht wurde, was sich darin zeigt, dass kein weiterer Sauerstoff mehr vom Reaktionsgemisch aufgenommen wird.

Bevorzugt wird die Umsetzung unter einem konstanten Sauerstoffüberdruck durchgeführt. Dies kann auf die Weise geschehen, dass in einem Druckreaktor über dem vorgelegten Mercaptoethanol, das mit dem Katalysator und mit Ammoniak und/oder mehreren Aminen versehen ist, eine Sauerstoffatmosphäre, z. B. mit einem sauerstoffhaltigen Gasgemisch mit einem bestimmten Überdruck eingestellt wird, beispielsweise 2,5 oder 5 bar. Der Reaktor steht in Verbindung mit einer Sauerstoffquelle, z. B. über ein mit einem Volumenmeßgerät versehenen Rohr. Durch dieses Rohr wird, solange die Reaktion dauert, Sauerstoff mit dem ausgewählten Überdruck nachgeliefert. Sobald die Reaktion zu Ende ist, findet kein Sauerstoffverbrauch und somit auch kein Nachliefern von Sauerstoff mehr statt.

Eine weitere Variante ist, dass man in dem Reaktionsgefäß einen bestimmten Ausgangsüberdruck einstellt, der sich infolge Verbrauchs von Sauerstoff entsprechend dem Reaktionsverlauf erniedrigt. Hat der Sauerstoff einen bestimmten niedrigeren Druckwert erreicht, wird durch neues Zuführen von Sauerstoff unter Druck wieder der Ausgangsdruck hergestellt. Dies wiederholt sich solange, bis der Druck konstant bleibt, d. h. dem Ausgangsüberdruck entspricht, was bedeutet, dass die Reaktion zu Ende geführt ist.

Eine weitere Ausführungsform des erfindungsgemäßen Verfahrens besteht darin, dass man nach dem Einfüllen des Mercaptoethanols in den Druckreaktor ein Inertgas z. B. Stickstoff einleitet. Sodann wird durch Zuführen von Sauerstoff unter Druck ein entsprechender Sauerstoffpartialdruck eingestellt, um ein Anspringen der Reaktion zu bewirken. Zur Vollendung der Reaktion wird die stöchiometrische Menge an Sauerstoff nachgeliefert.

Die Reaktion benötigt keine organischen Lösungsmittel, es ist ferner möglich, ohne Zusatz von Wasser zu arbeiten, d. h. der als Katalysator benötigte Ammoniak kann auch gasförmig dem Reaktionsgemisch beigegeben werden. Die Anwesenheit von Wasser stört allerdings die Reaktion nicht.

Als Amine können primäre, sekundäre oder auch tertiäre aliphatische Amine eingesetzt werden. Es ist auch möglich, Amingemische zu verwenden.

Als Amine können im Rahmen der Erfindung u. a. folgende Amine eingesetzt werden: Monomethylamin sowie Di- und Trimethylamin, Mono-, Di- und Triethylamin, Mono-, Di- und Tripropylamin und zwar sowohl die entsprechenden n-Propyl- als auch die Isopropylamine. Auch die entsprechenden Butylamine mit linearem oder verzweigtem Butylrest, d. h. die entsprechenden n-, iso- und t-Butylamine, sowie gemischte Amine wie beispielsweise Dimethylethylamin, Methylethylamin, Diethylmethylamin und dergleichen können verwendet werden. Die Verwendung weiterer aliphatischer Amine u. a. solche mit mehr als 4 Kohlenstoffatomen ist möglich.

Als Kupfersalze kommen sowohl 1- als auch 2-wertige Salze von organischen oder anorganischen Säuren in Frage. Als Beispiele für einwertige Kupfersalze sind zu nennen Kupfer-(I)-chlorid, -bromid, ₋jodid. Auch können Kupferrhodanide, -acetate, -sulfide usw. eingesetzt werden. Als Kupfer-(II)-salze sind Kupfer-(II)-chlorid, -bromid, -sulfid, -sulfat, -nitrat, -nitrit, - rhodanid, -cyanid usw. geeignet.

Als Mangansalze sind besonders geeignet Manganacetat, Mangansulfat und Manganchlorid sowie Mangansalze mit Anionen, wie sie vorstehend für die Kupfersalze genannt wurden.

Es war besonders überraschend, dass es mittels des erfindungsgemäßen Verfahrens möglich ist, Dihydroxiethylendisulfid durch Umsetzung von Mercaptoethanol mit Sauerstoff in hohen Ausbeuten herzustellen. Der Umsatz ist praktisch 100 %, das entstehende Produkt weist eine definierte einheitliche Zusammensetzung auf und ist wasserklar. Nebenreaktionen finden nicht statt, so dass kostspielige Reinigungsschritte entfallen.

So ist auch eine Rückgewinnung von nicht umgesetztem Ausgangsprodukt nicht erforderlich. Die Reaktionszeit ist sehr kurz. Da das Disulfid durch Destillation (Zersetzung) nicht gereinigt werden kann, ist dies ein besonderer Vorteil.

Das eingesetzte Amin bzw. der Ammoniak wird nach Abschluss der Reaktion zusammen mit gebildetem Reaktionswasser über Kopf abdestilliert und kann für die nächste Umsetzung wieder verwendet werden.

Da bei der Reaktion eine vollständige Umsetzung der eingesetzten Ausgangsprodukte stattfindet, sind auch keine besonderen Vorkehrungen für die Reinigung der Abluft erforderlich. Abwasserprobleme ergeben sich nicht.

Im Gegensatz zu den Verfahren gemäß dem Stand der Technik findet keine weitere Reaktion statt, wenn Sauerstoff im Überschuss vorhanden ist, was für die Produktionssicherheit ein weiterer großer Vorteil ist.

Der noch in Spuren im Reaktionsprodukt vorhandene Metallsalzkatalysator stört nicht. Das Endprodukt kann so direkt weiterverarbeitet werden.

Die Erfindung wird durch folgende Beispiele näher erläutert:

### Beispiel 1

In einem 100 1-Autoldaven werden 55 kg Mercaptoethanol, 200 ml konzentrierter Ammoniak und 15 mg Manganacetat vorgelegt. Der Reaktor ist mit einem Kühler und einem Begasungsrührer versehen. Der Begasungsrührer wird bei Raumtemperatur in Betrieb genommen und Sauerstoff über ein Flowmeter mit 2,0 bar aus einem Vorratsbehälter entnommen und das Ventil zum Autoklaven geöffnet.

Nach Erwärmen auf ca. 30° C springt die Reaktion an, was sich durch Verbrauch an Sauerstoff über das Flowmeter sowie das kräftige Ansteigen der Temperatur im Reaktor bemerkbar macht. Nun muss durch Kühlung, eventuell auch durch Reduzierung des Sauerstoffdrucks dafür Sorge getragen werden, dass die Temperatur 60° C möglichst nicht überschreitet. Sobald die stöchiometrische Menge Sauerstoff eingeleitet wurde, hierfür wird eine Zeit von 2 - 5 Stunden benötigt, hört die Reaktion von selbst auf, was durch Absinken der Temperatur im Reaktor und Aufhören des Gasflusses im Flowmeter erkennbar ist. Nun wird die Sauerstoffverbindung unterbrochen. Im Reaktor wird ein Vakuum von 50 - 100 mbar angelegt und Wasser zusammen mit Ammoniak über Kopf abdestilliert. Als Rückstand verbleiben 54 kg einer klaren Flüssigkeit, was einer Ausbeute von 99,5 % an Dihydroxiethylendisuldid entspricht.

### Beispiel 2

Analog Beispiel 1 werden 55 kg Mercaptoethanol zusammen mit 150 ml Triethylamin und 25 mg Kupfer(II)acetat vorgelegt und nach Einschalten des Begasungsrührers ein Sauerstoffdruck von 4 bar auf den Reaktor aufgedrückt und danach die Verbindung zu dem Sauerstoffvorratsgefäß wieder geschlossen.

Die Reaktion springt sofort an, die Temperatur steigt bis auf 60° C an, und der Sauerstoffdruck im Reaktor beginnt schnell abzufallen (Sauerstoffverbrauch). Wenn der Druck im Reaktor auf 1,5 bar abgefallen ist (ca. 30 min.), wird das Ventil zum Sauerstoffvorratsgefäß wieder geöffnet und damit der Druck im Reaktor wieder auf 4 bar erhöht. Erneut erfolgt eine merkliche Temperaturerhöhung auf 55 ° C, und der Druck fällt danach im Verlauf von ca. 30 Minuten wieder auf 1,5 bar ab.

Diese Vorgehensweise wird solange wiederholt, bis kein merklicher Druckabfall mehr feststellbar ist. Dies zeigt an, dass die Reaktion zum Ende gekommen ist und kein weiterer Sauerstoffverbrauch mehr stattfindet. Die Aufarbeitung erfolgt analog Beispiet 1 durch Anlegen von Vakuum und über Kopf-Destillation des Reaktionswassers zusammen mit dem tertiären Amin. Als Rückstand verbleiben 54,1 kg einer klaren leicht bräunlich gefärbten (durch die Spuren der im Produkt verbliebenen Kupfersalze) Flüssigkeit mit einer Reinheit von 99,9 %, was einer Ausbeute von 99,6 % an Dihydroxiethylendisuldid entspricht.

### Beispiel 3

In einem 800 Liter emaillierten Druckreaktor der Firma Pfaudler, der mit einem Düsenkranz, Rührer, Strömungsbrecher und Druckkühler ausgestattet ist, wurden 440 kg Mercaptoethanol zusammen mit 11 Tributylamin und 200 mg Mangansulfat vorgelegt und der Rührer in Betrieb genommen. Bei Raumtemperatur wird sodann komprimierte Luft mit 10 - 15 bar durch den Düsenkranz am Boden des Reaktors eingeleitet und über ein auf 8 - 10 bar eingestelltes Rückschlag (Überdruck-) ventl am Ende des Druckkühlers wieder ausgeleitet. Dabei wird ein Teil des Sauerstoffgehaltes der komprimierten Luft verbraucht, was sich durch einen Anstieg der Reaktionstemperatur im Reaktor bemerkbar macht. Die Menge der durchgeleiteten Druckluft wird so eingestellt, dass die Reaktionstemperatur im Reaktor 60° C nicht überschreitet.

Am Beginn der Reaktion ist deshalb die Luftzufuhr etwas zu drosseln, gegen Ende der Reaktion wird dagegen die volle Luftmenge (des Kompressors) durchgeleitet. Die Reaktionstemperatur sinkt nach ca. 8 h trotzdem wieder auf Raumtemperatur ab; die Umsetzung ist beendet. Nach Abtrennung von Reaktionswasser und Amin hinterbleiben 420 kg einer klaren Flüssigkeit entsprechend einer Ausbeute von 99 %. Ein kleiner Anteil an Mercaptoethanol wird mit der Luft ausgetragen und findet sich in der Kühlervorlage wieder, so dass die Selektivität der Reaktion praktisch quantitativ ist.

## Patentansprüche

1. Verfahren zur Herstellung von Dihydroxiethylendisuldid durch Umsetzung von Mercaptoethanol mit Sauerstoff, bei dem Mercaptoethanol in Gegenwart von Ammoniak und/oder Aminen unter Verwendung von Kupfer- oder Mangan-Salzen mit Sauerstoff oder einem sauerstoffhaltigen Gas umgesetzt wird.

2. Verfahren nach Anspruch 1, **dadurch gekennzeichnet, dass** man als Kupfersalz Kupfer-II-Salze verwendet.

3. Verfahren nach Anspruch 1, **dadurch gekennzeichnet, dass** man als Mangan-Salz Mangan-II-Salze verwendet.

4. Verfahren nach Anspruch 2, **dadurch gekennzeichnet, dass** man als Kupfer-II-Salz Kupferacetat verwendet.

5. Verfahren nach Anspruch 3, **dadurch gekennzeichnet, dass** man als Mangan-II-Salz Manganacetat verwendet.

6. Verfahren nach mindestens einem der Ansprüche 1- 5, **dadurch gekennzeichnet, dass** man Mercaptoethanol vorlegt und Sauerstoff in die Vorlage dosiert.

7. Verfahren nach mindestens einem der Ansprüche 1 - 6, **dadurch gekennzeichnet, dass** man die Reaktion unter einem Sauerstoffpartialdruck von 0,5 bis 5 bar durchführt.

8. Verfahren nach mindestens einem der Ansprüche 1 - 7, **dadurch gekennzeichnet, dass** man die Reaktion unter einem Sauerstoffüberdruck, von 1 - 5 bar durchführt.

9. Verfahren nach Anspruch 8, **dadurch gekennzeichnet, dass** man die Reaktion bei einem Druck von 1 - 2,5 bar durchführt.

10. Verfahren nach mindestens einem der Ansprüche 1- 9, **dadurch gekennzeichnet, dass** man die Reaktion unter einem konstanten Sauerstoffdruck durchführt.

11. Verfahren nach mindestens einem der Ansprüche 1- 10, **dadurch gekennzeichnet, dass** man für die Reaktion eine stöchiometrische Menge an Sauerstoff verwendet.

12. Verfahren nach mindestens einem der Ansprüche 1- 11, **dadurch gekennzeichnet, dass** man wässrigen Ammoniak verwendet.

13. Verfahren nach mindestens einem der Ansprüche 1 - 12, **dadurch gekennzeichnet, dass** man das Metallsalz in Ammoniak oder in dem Amin gelöst verwendet.

14. Verfahren nach mindestens einem der Ansprüche 1 -13, **dadurch gekennzeichnet, dass** man die Umsetzung bei 20 bis 60° C durchführt.

15. Verfahren nach Anspruch 14, **dadurch gekennzeichnet, dass** man die Umsetzung bei 35 bis 45° C durchfülrt.

16. Verfahren nach mindestens einem der Ansprüche 1-15, **dadurch gekennzeichnet, dass** man die Umsetzung in Abwesenheit von organischen Lösungsmitteln durchführt.

17. Verfahren nach mindestens einem der Ansprüche 1-16, **dadurch gekennzeichnet, dass** man die Umsetzung in einem wässrigen Milieu durchführt.

18. Verfahren nach Anspruch 17, **dadurch gekennzeichnet, dass** man die Umsetzung in Gegenwart von 10 - 70 Ges.-% Wasser bezogen auf die Gesamtmenge der eingesetzten Stoffe verwendet.

## Claims

1. Process for the preparation of dihydroxiethylene disulfide by reaction of mercaptoethanol with oxygen, wherein mercaptoethanol is reacted in the presence of ammonia and/or amines using copper or manganese salts with oxygen or an oxygen-containing gas.

2. Process according to claim 1, **characterised in that** as copper salt copper(II) salt is used.

3. Process according to claim 1, **characterised in that** as manganese salt manganese(II) salt is used.

4. Process according to claim 2, **characterised in that** as copper(II) salt copper acetate is used.

5. Process according to claim 3, **characterised in that** as mangenese(II) salt manganese acetate is used.

6. Process according to at least one of claims 1-5, **characterised in that** mercaptoethanol is placed in a receiver and oxygen is dosed into the receiver.

7. Process according to at least one of claims 1-6, **characterised in that** the reaction is carried out under an oxygen partial pressure of 0.5 to 5 bar.

8. Process according to at least one of claims 1 -7, **characterised in that** the reaction is carried out under an oxygen excess pressure of 1-5 bar.

9. Process according to claim 8, **characterised in that** the reaction is carried out at a pressure of 1-2.5 bar.

10. Process according to at least one of claims 1-9, **characterised in that** the reaction is carried out under a constant oxygen pressure.

11. Process according to at least one of claims 1-10, **characterised in that** a stoichiometric amount of oxygen is used for the reaction.

12. Process according to at least one of claims 1-11, **characterised in that** aqueous ammonia is used.

13. Process according to at least one of claims 1-12, **characterised in that** the metal salt is used dissolved in ammonia or in amine.

14. Process according to at least one of claims 1- 13, **characterised in that** the reaction is carried out at 20 to 60°C.

15. Process according to claim 14, **characterised in that** the reaction is carried out at 35 to 45°C.

16. Process according to at least one of claims 1-15, **characterised in that** the reaction is carried out in the absence of organic solvents.

17. Process according to at least one of claims 1-16, **characterised in that** the reaction is carried out in an aqueous environment.

18. Process according to claim 17, **characterised in that** the reaction is carried out in the presence of 10-70 wt.% of water, based on the total amount of substances used.

## Revendications

1. Procédé de production de disulfure de dihydroxyéthylène par réaction de mercaptoéthanol avec de l'oxygène, dans lequel on fait réagir du mercaptoéthanol en présence d'ammoniaque et/ou d'amines en utilisant des sels de cuivre ou de manganèse avec de l'oxygène ou un gaz contenant de l'oxygène.

2. Procédé selon la revendication 1, **caractérisé en ce que** l'on utilise comme sel de cuivre, du sel de cuivre II.

3. Procédé selon la revendication 1, **caractérisé en ce que** l'on utilise comme sel de manganèse, du sel de manganèse II.

4. Procédé selon la revendication 2, **caractérisé en ce que** l'on utilise comme sel de cuivre II, de l'acétate de cuivre.

5. Procédé selon la revendication 3, **caractérisé en ce que** l'on utilise comme sel de manganèse II, de l'acétate de manganèse.

6. Procédé selon au moins l'une des revendications 1 à 5, caractérisé en ce l'on charge du mercaptoéthanol et l'on ajoute de l'oxygène dans le récipient.

7. Procédé selon au moins l'une des revendications 1 à 6, **caractérisé en ce que** l'on réalise la réaction sous une pression partielle d'oxygène de 0,5 à 5 bars.

8. Procédé selon au moins l'une des revendications 1 à 7, **caractérisé en ce que** l'on réalise la réaction sous une surpression d'oxygène de 1 à 5 bars.

9. Procédé selon la revendication 8, **caractérisé en ce que** l'on réalise la réaction à une pression de 1 à 2,5 bars.

10. Procédé selon au moins l'une des revendications 1 à 9, **caractérisé en ce que** l'on réalise la réaction sous une pression d'oxygène constante.

11. Procédé selon au moins l'une des revendications 1 à 10, **caractérisé en ce que** l'on utilise pour la réaction, une quantité stoechiométrique d'oxygène.

12. Procédé selon au moins l'une des revendications 1 à 11, **caractérisé en ce que** l'on utilise de l'ammoniaque aqueuse.

13. Procédé selon au moins l'une des revendications 1 à 12, **caractérisé en ce que** l'on utilise le sel de métal dissous dans de l'ammoniaque ou dans l'amine.

14. Procédé selon au moins l'une des revendications 1 à 13, **caractérisé en ce que** l'on réalise la réaction de 20 à 60° C.

15. Procédé selon la revendication 14, **caractérisé en ce que** l'on réalise la réaction de 35 à 45° C.

16. Procédé selon au moins l'une des revendications 1 à 15, **caractérisé en ce que** l'on réalise la réaction en l'absence de solvants organiques.

17. Procédé selon au moins l'une des revendications 1 à 16, **caractérisé en ce que** l'on réalise la réaction dans un milieu aqueux.

18. Procédé selon la revendication 17, **caractérisé en ce que** l'on réalise la réaction en présence de 10 à 70 % en poids d'eau par rapport à la quantité totale de matériau utilisé.
